# EUROPEAN PATENT APPLICATION

(11) **EP 3 693 456 A1**
(43) Date of publication of application: **12.08.2020**
(21) Application number: 18864957.8
(22) Date of filing: 04.10.2018
(51) Int. Cl.: C12N 5/0783, A61K 35/17, A61K 35/545, A61P 7/00, A61P 35/00, A61P 37/04, A61P 43/00, C12N 5/10, C12N 15/09

(54) **PRODUCTION METHOD FOR IPS CELL-DERIVED GENETICALLY DIVERSE T CELL COLONY**

(30) Priority: 06.10.2017 JP 2017196289
(71) Applicant: Thyas Co. Ltd., Kyoto-shi, Kyoto 606-8304 (JP); Kyoto University, Kyoto-shi, Kyoto 606-8501 (JP)
(72) Inventor: YASUI Yutaka, Kyoto-shi Kyoto 606-8304 (JP); KATSURA Yoshimoto, Kyoto-shi Kyoto 606-8304 (JP); UENO Hiroyuki, Kyoto-shi Kyoto 606-8304 (JP); KANEKO, Shin, Kyoto-shi Kyoto 606-8304 (JP)
(74) Representative: Bassil, Nicholas Charles
(86) International application number: PCT/JP2018/037188
(87) International publication number: WO 2019/070021

(57) **Abstract**

[Problem] To provide a method for producing a population of genetically diverse regenerated T cells via iPS cells, and to provide said population of regenerated T cells.

[Solution] A method for producing a population of genetically diverse regenerated T cells via iPS cells, including: (1) obtaining a population of T cells that can recognize a target tissue or antigens from a population of the genetically diverse T cells; (2) reprogramming the obtained the population of T cells into iPS cells, culturing the iPS cells while maintaining genetic diversity; and (3) producing a population of the genetically diverse regenerated T cells from the iPS cells.

## Description

### TECHNICAL FIELD

The present invention relates to a method for producing a population of genetically diverse regenerated T cells and said population of regenerated T cells.

### TECHNICAL BACKGROUND

T cells play a key role in the immune system against foreign pathogens, such as bacteria or viruses, or abnormal cells, such as cancer cells. It is considered that subjects will become infectious or suffer from cancer when their T cell function declines for various reasons. If immune cells and the like can be replenished or regenerated, it will be an extremely effective means against these diseases to improve pathological conditions and therapeutic effects.

Replacement therapy using T cells derived from pluripotent stem cells such as iPS cells has been proposed. By using T cells which are specific for a target antigen as a raw material of iPS cells, regenerated T cells that show the same antigen specificity as the original T cells can be produced (Nonpatent document 1, The contents thereof are incorporated herein by reference.). In this method, established iPS cells are cloned in units of cell groups (colonies) derived from single cells. Accordingly, an iPS cell line, which has been confirmed to be stable in cell properties and highly efficient in differentiation into a target cell/tissue but without mutation or abnormality by gene analysis, or the like can be used. Since it is necessary to inspect clones one by one in order to confirm the quality and suitability for patients of the clones, cloning has conventionally been considered as an important process.

On the other hand, in studies using human and mouse subjects, when conducting T cell replacement therapy against infectious diseases and tumors by a method using genetically diverse T cells, diverse immunoreactions to a target tissue or antigen can be induced. At the same time, it is known that the method is less susceptible to immune escape due to a decrease in the expression or a mutation of an antigen, thus good therapeutic effects can be achieved.

Besides, there is a gene induction method of T cell receptor (TCR) or chimeric antigen receptor (CAR) to peripheral blood T cells in the prior art. However, the antigen receptor of T cells used has single gene sequences.

### PRIOR ART DOCUMENT

### Nonpatent document

1. Cell Stem Cell (2013) 12(1):114-126

### SUMMARY OF THE INVENTION

### Problems to be solved by the invention

Cloning is conducted in conventional methods utilizing iPS cell lines. However, this is equivalent to excluding non-selected clones. For this reason, the genetic diversity at the time of iPS cell establishment is completely lost. Accordingly, immunoreaction with a single antigenic epitope becomes available. However, the immunoreaction with a single antigenic epitope is susceptible to immune escape due to a decrease in expression and a mutation of an antigen, which causes a problem that good therapeutic effects can hardly be achieved. On the other hand, in the conventional T cell replacement therapy, a population of genetically diverse T cells can be used. However, T cells exhaust due to amplification of a population of T cells in vitro, which causes a problem of decrease in immunoreaction with antigen. Therefore, the development of a new method for producing a population of genetically diverse regenerated T cells are desired for use in T cell replacement therapy.

Accordingly, an object of the present invention is to provide a method for producing a population of genetically diverse regenerated T cells via iPS cells and said population of regenerated T cells. In another embodiment of the present invention, an object is to use the population of regenerated T cells obtained by the method described above in T cell replacement therapy. Moreover, in a further embodiment of the present invention, an object is to use tumor-infiltrating T cells as the T cells, which is a raw material in the method described above.

### Means for solving the problems

The present inventors have examined the problems in the conventional method and found out that if a population of genetically diverse regenerated T cells is produced via iPS cells, the problem that only an immunoreaction with a single antigen epitope is available and the problem of exhaustion of the population of T cells were solved. That is, the present invention is summarized as follows.
[1] A method for producing a population of genetically diverse regenerated T cells via iPS cells,
   The method comprising
   (1) obtaining a population of T cells that can recognize a target tissue or antigens from a sample of the population of genetically diverse T cells;
   (2) reprogramming the obtained population of T cells into iPS cells;
      culturing the iPS cells while maintaining genetic diversity; and
   (3) producing a population of genetically diverse regenerated T cells from the cultured iPS cells.
[2] The method according to [1], wherein the population of T cells in (1) are derived from a treated mammalian subject.
[3] The method according to [1] or [2], wherein the population of T cells in (1) are tumor-infiltrating T cells.
[4] The method according to [1] or [2], wherein the population of T cells in (1) is derived from blood, lymph nodes or cavity fluid.
[5] The method according to any one of [1] to [4], wherein (1) further comprises separating proliferated T cells, which are activated by stimulation with an antigen protein or peptides.
[6] The method according to any one of [1] to [5], comprising collecting iPS cells without cloning and subculturing the iPS cells in (2).
[7] The method according to any one of [1] to [6], wherein the population of genetically diverse regenerated T cells obtained in (3) is a population of αβ T cells, a population of γδ T cells, a population of helper T cells, a population of regulatory T cells, a population of cytotoxic T cells, a population of NK T cells or a population of tumor-infiltrating T cells.
[8] The method according to any one of [1] to [7], wherein the population of genetically diverse regenerated T cells obtained in (3) are used for T cell replacement therapy.
[9] A population of regenerated T cells obtained by the method according to any one of [1] to [8].
[10] A population of regenerated T cells obtained via iPS cells that maintains genetic diversity of the population of T cells present *in vivo.*
[11] A pharmaceutical composition, comprising the population of the regenerated T cells according to [9] or [10].
[12] The pharmaceutical composition according to [11] for treating cancer subjects by autologous or allogeneic transplantation.
[13] A method for treating cancers, which uses the pharmaceutical composition according to [11] or [12].

### EFFECTS OF THE INVENTION

According to the present invention, a population of genetically diverse regenerated T cells can be produced via iPS cells. Furthermore, in another embodiment of the present invention, a population of regenerated T cells produced from a population of *in vivo* genetically diverse T cells by selecting and using T cells that can recognize a target tissue or antigens can show a good therapeutic effect in T cell replacement therapy. Particularly, tumor-infiltrating T cells (tumor-infiltrating lymphocytes, TILs) are known to be specific for tumors and recognize a variety of antigens. By producing regenerated TILs via reprogramming from TILs to iPS cells, tumor-specific and effective T cell replacement therapy caused by genetically diverse rejuvenated T cells can be achieved.

### BRIEF DESCRIPTION OF THE FIGURES

Figure 1 shows the proportion of the T cells having a Vβ chain of each TCR in the population of T cells separated from resected tumor of a lung cancer patient. Vβ1 ∼ Vβ23 in Figure 1 indicate that the population of T cells separated are a population of T cells having a genetic diversity of TCR.
Figure 2 shows the proportion of the T cells having a Vβ chain of each TCR in the population of regenerated T cells produced via iPS cells. Vβ1 ∼ Vβ23 in Figure 2 indicate that the population of regenerated T cells are also a population of genetically diverse T cells.

### EMBODIMENTS FOR CARRYING OUT THE INVENTION

The present invention provides a method for producing a population of genetically diverse regenerated T cells via iPS cells. The method comprises (1) a step in which a population of T cells that can recognize a target tissue or antigens are selected from a population of genetically diverse T cells; (2) a step in which the selected population of T cells are reprogrammed into iPS cells and cultured, while maintaining genetic diversity; and (3) a step in which a population of genetically diverse regenerated T cells are produced from the cultured iPS cells.

In an embodiment of the present invention, "producing a population of genetically diverse regenerated T cells via iPS cells" refers to regenerating a population of genetically diverse T cells from a population of genetically diverse T cells via reprogramming to iPS cells. In the present invention, the T cells produced in the above process are referred to as "regenerated T cells".

### Step (1)

Step (1) of the present invention is a step in which a population of T cells that can recognize a target tissue or antigens is selected from a sample of a population of genetically diverse T cells.

### A population of genetically diverse T cells

In an embodiment of the present invention, "a population of genetically diverse T cells" refers to a lymphocyte whose *in vivo* CD3 and CD45 are positive, and various colonies wherein gene sequences of T cell receptor (TCR) that recognize antigens are in groups. Regarding *in vivo* T cells, when T precursor cells develop and differentiate in the thymus, random recombination of the TCR gene occurs. Accordingly, each T cell has a different TCR gene sequence, which may trigger immunoreactions with various antigens. Antigen or peptide sequences specifically recognized by TCR in individual T cell is determined. However, by considering the T cells, it can be understood that a population of the T cells is immunized with various antigens. Therefore, a population of T cells sampled from a living body is, in this sense, a population of genetically diverse T cells.

In an embodiment of the present invention, T cells are derived preferably from a mammal, more preferably from a human being, and even more preferably from a mammal (preferably a human being) of treated subject. Examples of T cells include, but are not particularly limited to, αβ T cells, γδ T cells, helper T cells, regulatory T cells, cytotoxic T cells, NK T cells, and the like. Besides, as a source of T cells, peripheral blood is preferable because of its low invasiveness. Examples of other preferable sources include cancer, tumor, other organs or tissues, cavity fluid (pleural effusion or ascites, etc.), body fluid (blood, etc.) and any *in vivo* source such as lymph nodes or cord blood, etc. In another embodiment of the present invention, preferable T cells are tumor-infiltrating T cells.

In an embodiment of the present invention, a population of genetically diverse T cells which react with any antigen or tissue can be used. Particularly, in a case that a population of regenerated T cells produced are used in T cell replacement therapy, the T cells sampled preferably can recognize the cancer, tumor, cancer antigens, tumor-specific mutant antigens, virus, or the like to be treated. Examples of such cancer or tumor to be treated include preferably any cancer or tumor that may occur *in vivo,* such as a solid tumor (lung cancer or gastric cancer, etc.), leukemia (acute or chronic myeloid leukemia or lymphocytic leukemia), or lymphoma, etc. Furthermore, examples of such cancer antigens, tumor-specific mutant antigens, or viruses include preferably cancer antigens which specifically or non-specifically express in each tumor such as WT1, MUC1, EGFRvIII, HER-2/neu, MAGE A3, p53 nonmutant, NY-ESO-1, PSMA, GD2, CEA, MelanA/MART1, or Survivin, etc., those accompanied with gene mutation such as p53, Ras, ERG, bcr-abl, neoantigen, or proteins derived from various viruses such as LMP2, HPV E6 or E7, EB-NA, or HTLV-1 Tax, etc.

### The step in which a population of T cells that can recognize a target tissue or antigens are selected

There are a great variety of T cells in a living body so that they can respond to any antigen. However, most of them are unrelated to the antigen of interest in T cell replacement therapy. Therefore, as a raw material for producing T cells via a step of reprogramming to iPS cells, a population of T cells that are reactive to cancer, tumor or virus targeted for T cell replacement therapy is previously selected, so that the therapeutic effect can be dramatically improved, and at the same time, unexpected immunoreactions with antigens are suppressed so that the safety can be improved.

In an embodiment of the present invention, "target tissue or antigen" refers to preferably a tissue comprising a cancer, a tumor, a cancer antigen, a tumor-specific mutant antigen, virus of a subject to be treated as described above or an antigen contained therein.

In an embodiment of the present invention, "can recognize" refers to that an individual T cell shows an immunoreaction with a target tissue or antigen as a group, while specifically recognizing different antigen or peptide sequences.

In an embodiment of the present invention, as an example of methods for selecting a population of T cells that can recognize a target tissue or antigens, the population of T cells are activated by stimulating with an antigen protein or peptides, and proliferated T cells are separated. In this method, a monocyte fraction containing antigen-presenting cells is cultured, and antigen protein or peptide is added to culture medium. Reactive T cells proliferate due to repetitive stimulations with antigen protein or peptide, and their proportions in the population of T cells increase. On the other hand, T cells that do not react with the antigenic protein or peptide added are not activated, the proportions thereof decrease during the *in vitro* culturing gradually, and eventually die. Therefore, after culturing for a certain period, a population of T cells is obtained which has specific reactivity with the antigen protein or peptide added and diversity in recognizing antigen or peptide sequence. As the "antigen protein or peptide", those described above as the target tissue or antigen are preferable. For example, peptide fragments of cancer antigens, tumor-specific mutant antigens or viruses as described above can be used. In addition, T cells stimulated by an antigen peptide are known to express or release various activating molecules or cytokines. In an embodiment of the present invention, utilizing this property, an antibody specific to those molecules can be provided to magnetic separation using magnetic beads or selective separation using a flow cytometer with fluorescence labeling after binding to those molecules. Besides, in an embodiment of the present invention, a system for capturing cytokine-releasing cells with a bipolar antibody (on cell side and cytokine side) can be used.

In another embodiment of the present invention, as an example of methods for selecting a population of T cells that can recognize d a target tissue or antigens, an oligomer of an antigen peptide-HLA complex, such as a tetramer or dextramer is used. In this method, an oligomer consisting of a complex of a specific HLA type and a target antigen peptide capable of presenting the HLA is labeled with a fluorescent dye. The cells to which the oligomer is bound are sorted using a flow cytometer. Accordingly, T cells having tropism toward a target tissue or antigen can be obtained.

### Step (2)

Step (2) of the present invention is a step in which the selected population of T cells is reprogrammed into iPS cells and cultured while maintaining genetic diversity.

### The step in which the selected population of T cells is reprogrammed into iPS cells

Methods for producing iPS cells are known in the art. In an embodiment of the present invention, iPS cells are preferably produced by introducing a reprogramming factor into the population of T cells selected in step (1). Examples of the reprogramming factors include genes or gene products, such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, Glis1, and the like. These reprogramming factors can be used alone or in combination.

### The step in which established iPS cells are cultured while maintaining genetic diversity

When culturing iPS cells, it is preferable to conduct subculture. However, in a common subculture method, cells except those used for the subculture are discarded. Accordingly, the diversity of iPS cells established from the population of genetically diverse T cells are gradually lost.

In contrast, in an embodiment of the present invention, preferably, after establishing iPS cells, the incubator is washed. Preferably, cells except iPS cells are removed. iPS cells are collected without cloning and subcultured in another incubator. Such subculture is repeated.

In an embodiment of the present invention, the culture medium used in this step is not particularly limited. It can be prepared by using a medium useful for culturing animal cells as a minimal essential medium and adding cytokines thereto for maintaining anaplastic ability of iPS cells. Examples of minimal essential media include Iscove's Modified Dulbecco's Medium (IMDM), Medium 199, Eagle's minimum essential medium (EMEM), αMEM medium, Dulbecco's modified Eagle's medium (DMEM), Ham's F12 medium, RPMI 1640 medium, Fischer's medium, Neurobasal Medium (Life Technologies), StemFit AK03N (Ajinomoto) and a mixed medium thereof. A medium may contain serum or may be serum-free. A preferable example of cytokines is bFGF. Its concentration in a culture medium is, for example, 1∼100 µg/mL (preferably 50 µg/mL).

In an embodiment of the present invention, methods for culturing iPS cells may be adhesion culture or suspension culture, preferably the adhesion culture. Examples of methods for separating iPS cells include a mechanical separation method, and separation methods using a separation solution having protease activity, a separation solution having collagenase activity, or a separation solution having protease activity and collagenase activity (Accutase™ and Accumax™, etc.).

In an embodiment of the present invention, iPS cells are subcultured in another incubator when they reach a cell density of preferably 1x10³ ∼ 1x10⁴ cells/cm², 1x10⁴ ∼ 1x10⁵ cells/cm², 1x10⁵ ∼ 1x10⁶ cells/cm². The times of subculture may be any times as long as iPS cells are obtained in an amount required for a T cell replacement therapy, preferably 1 ∼ 5 times or 5 ∼ 10 times.

In an embodiment of the present invention, the genetically diverse iPS cells obtained may be used as they are or may be cryopreserved until needed.

### Step (3)

Step (3) of the present invention is a step in which a population of genetically diverse regenerated T cells is produced from the cultured iPS cells.

In an embodiment of the present invention, preferably, a population of genetically diverse regenerated T cells is obtained by differentiating the iPS cells cultured in step (2) into CD4CD8 double positive T cells via hematopoietic progenitor cells, or by differentiating the iPS cells cultured in step (2) into CD8 positive T cells via these cells.

### The step in which hematopoietic progenitor cells are induced from iPS cells

Hematopoietic progenitor cells (HPC) are cells that can be differentiated into hematopoietic cells such as lymphocytes, eosinophils, neutrophils, basophils, erythrocytes, or megakaryocytes, etc. In an embodiment of the present invention, hematopoietic progenitor cells and hematopoietic stem cells are not distinguished and are referred to the same cells unless otherwise specified. Hematopoietic stem/progenitor cells can be recognized, for example, if surface antigens CD34 and/or CD43 are positive.

In an embodiment of the present invention, hematopoietic progenitor cells are preferably produced by culturing iPS cells in a culture medium added with vitamin Cs. The "vitamin Cs" refer to L-ascorbic acid and its derivatives. "An L-ascorbic acid derivative" refers to something that turns into vitamin C in an enzymatic reaction *in vivo.* Examples of L-ascorbic acid derivatives include vitamin C phosphate, glucoside ascorbate, ascorbyl ethyl, vitamin C ester, ascorbyl tetrahexyldecanoate, ascorbyl stearate and ascorbic acid-2 phosphoric acid-6 palmitic acid. A preferable L-ascorbic acid derivative is vitamin C phosphate, for example, a phosphoric acid-L ascorbate, like phosphoric acid sodium-L-ascorbate or phosphoric acid magnesium-L-ascorbate. Vitamin Cs are contained in a culture medium at a concentration of, for example, 5 (µg/ml ∼ 500 µg/ml.

In an embodiment of the present invention, the culture medium for producing hematopoietic progenitor cells is not particularly limited. It can be prepared by using a medium useful for culturing animal cells as a minimal essential medium and adding vitamin Cs etc. thereto. Examples of minimal essential media include Iscove's Modified Dulbecco's Medium (IMDM), Medium 199, Eagle's minimum essential medium (EMEM), αMEM medium, Dulbecco's modified Eagle's Medium (DMEM), Ham's F12 medium, RPMI 1640 medium, Fischer's medium, Neurobasal Medium (Life Technologies), StemPro34 (Life Technologies) and a mixed medium thereof. A medium may contain serum or may be serum-free. If necessary, a minimal essential medium may contain one or more substances, for example, albumin, insulin, transferrin, selenium, fatty acids, trace elements, 2-mercaptoethanol, thiolglycerol, lipids, amino acids, L-glutamine, non-essential amino acids, vitamins, growth factors, low molecular compounds, antibiotics, antioxidants, pyruvic acid, buffers, inorganic salts, cytokines, and the like.

In an embodiment of the present invention, the culture medium for producing hematopoietic progenitor cells may be furtherly added with a cytokine selected from the group consisting of BMP4 (Bone morphogenetic protein 4), VEGF (vascular endothelial growth factor), bFGF (basic fibroblast growth factor), SCF (Stem cell factor), TPO (Thrombopoietin) and FLT-3L (Flt3 Ligand).

In an embodiment of the invention, their concentrations are, for example, 1 ng/ml ∼ 100 ng/ml for BMP4, 1 ng/ml ∼ 100 ng/ml for VEGF, and 1 ng/ml - 100 ng/ml for bFGF, 10 ng/ml - 100 ng/ml for SCF, 1 ng/ml - 100 ng/ml for TPO, and 1 ng/ml - 100 ng/ml for FLT-3L.

In an embodiment of the present invention, a TGFβ inhibitor may be added to a culture medium. "A TGFβ inhibitor" refers to a small molecule inhibitor that interferes with TGFβ family signaling, such as SB431542, SB202190 (R. K. Lindemann et al., Mol. Cancer 2: 20 (2003), for the two inhibitors), SB505124 (GlaxoSmithKline), NPC30345, SD093, SD908, SD208 (Scios), LY2109761, LY364947 and LY580276 (Lilly Research Laboratories), etc. The concentrations thereof in a medium are preferably 0.5 µM ∼ 100 µM.

In an embodiment of the present invention, the iPS cells may be cultured with feeder cells, such as C3H10T1/2 (Takayama N., et al. J Exp Med. 2817-2830, 2010) or xenogeneic stromal cells (Niwa A., et al. J Cell Physiol. 2009 Nov; 221 (2): 367-77.), etc. However, they are preferably cultured without using feeder cells.

In an embodiment of the present invention, the method for culturing iPS cells during the production of hematopoietic progenitor cells may be adhesion culture or suspension culture, preferably the suspension culture. For example, iPS cells can be subjected to suspension culture after separating a colony cultured to 80% confluent with the used dish and dissociating it into single cells. Examples of methods for separating iPS cells include a mechanical separation method, and separation methods using a separation solution having protease activity and collagenase activity (Accutase™ and Accumax™, etc.), or a separation solution having collagenase activity.

Suspension culture refers to culturing cells without adhesion with the culture vessel. In an embodiment of the present invention, the suspension culture is not particularly limited. However, for the purpose of improving the adhesiveness to cells, a culture vessel which is not artificially treated (for example, a coated with an extracellular matrix etc.) or a culture vessel treated for suppressing the adhesiveness (e.g., coated with polyhydroxyethyl methacrylic acid (poly-HEMA) or a nonionic surfactant polyol (Pluronic F-127, etc.) can be used for the culturing. In the case of suspension culture, it is preferable to form an embryoid body (EB).

In another embodiment of the present invention, hematopoietic progenitor cells can be prepared from a net-like structure (also referred to as iPS-sac) obtained by culturing iPS cells. "A net-like structure" refers to a three-dimensional sac-like structure (having inner space) which is derived from iPS cells, formed in an endothelial cell colony or the like and contains hematopoietic progenitor cells inside.

In an embodiment of the present invention, in order to produce hematopoietic progenitor cells, the temperature for culturing is not particularly limited. However, for example, temperatures in the ranges of about 37°C ∼ about 42°C and about 37°C ∼ about 39°C are preferable. Furthermore, those skilled in the art can appropriately determine the culture period while monitoring the number of hematopoietic progenitor cells, and the like. As long as hematopoietic progenitor cells can be obtained, the number of days is not particularly limited, and may be, for example, at least 6 days or more, 7 days or more, 8 days or more, 9 days or more, 10 days or more, 11 days or more, 12 days or more, 13 days or more and 14 days or more, preferably 14 days. A long culture period is not problematic in the production of hematopoietic progenitor cells. Besides, the culturing may be conducted under low oxygen conditions. In an embodiment of the present invention, examples of the low oxygen conditions include oxygen concentrations of 15%, 10%, 9%, 8%, 7%, 6%, 5% or less.

### The step in which CD4CD8 double positive T cells are induced from hematopoietic progenitor cells

In the present invention, the "CD4-CD8 double positive T cells" refer to the cells (CD8⁺ CD4⁺) whose surface antigens both CD4 and CD8 are positive among T cells. Since T cells can be recognized by their surface antigens CD3 and CD45 being positive, CD4CD8 double positive T cells can be identified as the cells whose CD4, CD8, CD3 and CD45 are positive. CD4CD8 double positive T cells can be differentiated by induction into CD4 positive cells or CD8 positive cells.

In an embodiment of the present invention, CD4CD8 double positive T cells can be produced by a method comprising a step in which hematopoietic progenitor cells are cultured in a culture medium added with a p38 inhibitor and/or SDF-1.

### [p38 inhibitors]

In the present invention, the "p38 inhibitors" are defined as a substance that inhibits the function of p38 protein (p38MAP kinase). In an embodiment of the present invention, their examples include, but are not limited to, a chemical inhibitor of p38, a dominant negative mutant of p38, a nucleic acid encoding the same, and the like.

In an embodiment of the present invention, examples of chemical inhibitors of p38 include, but are not limited to, SB203580 (4-(4-fluorophenyl)-2-(4-methylsulfonyl phenyl)-5-(4-pyridyl)-1H-imidazole) and its derivatives, SB202190 (4-(4-fluorophenyl)-2-(4-hydroxyphenyl)-5-(4-pyridyl)-1H-imidazole) and its derivatives, SB239063 (trans-4-[4-(4-fluorophenyl)-5-(2-methoxy-4-pyrimidinyl)-1H-imidazol-1-yl]cyclohexanol) and its derivatives, SB220025 and its derivatives, PD169316, RPR200765A, AMG-548, BIRB-796, SC10-469, SCIO-323, VX-702 and FR167653. These compounds are commercially available. For example, SB203580, SB202190, SC239063, SB220025 and PD169316 can be purchased from Calbiochem, while SC10-469 and SCIO-323 can be purchased from Scios.

In an embodiment of the present invention, examples of dominant negative mutants of p38 include p38T180A in which the threonine at position 180 located in the DNA binding region of p38 has been point-mutated to alanine and p38Y182F in which the tyrosine at position 182 of p38 in a human being or a mouse has been point-mutated to phenylalanine, etc.

A p38 inhibitor is contained in a medium at a concentration ranging, for example, from about 1 µM ∼ about 50 µM.

### [SDF-1]

In an embodiment of the present invention, SDF-1 (Stromal cell-derived factor 1) is not just SDF-1α or its mature form. It may also be an iso form such as SDF-1β, SDF-1γ, SDF-1δ, SDF-1ε or SDF-1ϕ, etc. or a mature form thereof, or a mixture thereof in any proportion, among which SDF-1α is preferably used. Besides, SDF-1 can be referred to as CXCL-12 or PBSF.

In an embodiment of the present invention, as long as SDF-1 has the activity as a chemokine, one or more amino acids in its amino acid sequence may be substituted, deleted and/or added. Similarly, its sugar chains may also be substituted, deleted and/or added. At least four cysteine residues (Cys30, Cys32, Cys55 and Cys71 in the case of human SDF-1α) are retained in SDF-1. If it also has 90% or more identity with the amino acid sequence of a natural form, amino acid mutations are allowed. SDF-1 may be from a mammal, such as a human being, or from a non-human mammal, such as a monkey, sheep, cow, horse, pig, dog, cat, rabbit, rat, or mouse, etc. For example, a protein registered under GenBank accession number: NP_954637 can be used as human SDF-1α, and a protein registered under GenBank accession number: NP_000600 can be used as SDF-1β.

In an embodiment of the present invention, as SDF-1, a commercially available product and those purified from nature or produced by peptide synthesis or by using genetic engineering techniques can be used.

In an embodiment of the present invention, SDF-1 is contained in a medium at a concentration ranging, for example, from about 10 ng/ml to about 100 ng/ml.

In an embodiment of the present invention, the culture medium for producing CD4CD8 double positive T cells is not particularly limited. It can be prepared by using a medium useful for culturing animal cells as a minimal essential medium and adding a p38 inhibitor and/or SDF-1, more preferably, vitamin Cs. The types of vitamin Cs used in the production process of CD4CD8 double positive T cells are, for example, those described above. The concentrations of vitamin Cs are, for example, 5 µg/ml ∼ 200 µg/ml. Examples of minimal essential media include Iscove's Modified Dulbecco's Medium (IMDM), Medium 199, Eagle's Minimum Essential Medium (EMEM), αMEM medium, Dulbecco's modified Eagle's Medium (DMEM), Ham's F12 medium, RPMI 1640 medium, Fischer's medium, OP9 medium, Neurobasal Medium (Life Technologies) and a mixed medium thereof. A medium may contain serum or may be serum-free. If necessary, a minimal essential medium may contain one or more substances, for example, albumin, insulin, transferrin, selenium, fatty acids, trace elements, 2-mercaptoethanol, thiolglycerol, lipids, amino acids, L-glutamine, non-essential amino acids, vitamins, growth factors, low molecular compounds, antibiotics, antioxidants, pyruvic acid, buffers, inorganic salts, cytokines, and the like.

In an embodiment of the present invention, the culture medium for producing CD4CD8 double positive T cells can be furtherly added with a cytokine selected from the group consisting of SCF, TPO (Trombopoietin), FLT-3L and IL-7. Their concentrations are, for example, 10 ng/ml ∼ 100 ng/ml for SCF, 10 ng/ml ∼ 200 ng/ml for TPO, 1 ng/ml ∼ 100 ng/ml for FLT-3L and 1 ng/ml ∼ 100 ng/ml for IL-7.

In an embodiment of the present invention, when producing CD4CD8 double positive T cells, hematopoietic progenitor cells may be cultured using feeder cells. However, they are preferably cultured without using feeder cells.

In an embodiment of the present invention, hematopoietic progenitor cells may be adhesion cultured or suspension cultured, although adhesion culture is preferable in this step. In case of the adhesion culture, a culture vessel may be coated. Examples of coating agents include Matrigel (Niwa A, et al. PLoS One.6 (7): e22261, 2011), collagen, gelatin, laminin, heparan sulfate proteoglycans, retronectin, Fc-DLL4 or entactin and combinations thereof.

In another embodiment of the present invention, when the embryoid body is suspension cultured to obtain hematopoietic progenitor cells, it is preferable to dissociate it into single cells before conducting an adhesion culture.

In an embodiment of the present invention, in order to produce CD4CD8 double positive T cells, the temperature for culturing hematopoietic progenitor cells is not particularly limited. However, for example, a temperature in the ranges of about 37°C ∼ about 42°C, and about 37°C ∼ about 39°C is preferable. In addition, those skilled in the art can appropriately determine the culture period while monitoring the number of CD4CD8 double positive T cells, and the like. As long as CD4CD8 double positive T cells can be obtained, the number of days is not particularly limited, for example, at least 10 days or more, 12 days or more, 14 days or more, 16 days or more, 18 days or more, 20 days or more, 22 days or more, 23 days or more, preferably 23 days.

In an embodiment of the present invention, the CD4CD8 double positive T cells obtained may be separated before use, or may be used as a cell colony comprised of other cell strains. In case of separation, it can be separated using any one of the indicators consisting of CD4, CD8, CD3 and CD45. A method well known to those skilled in the art can be used as the separation method, for example, a separation method using a flow cytometer after labeling with antibodies of CD4, CD8, CD3 and CD45, or a purification method using an affinity column fixed with desired antigen.

### The step in which CD8 positive T cells are induced from CD4CD8 double positive T cells

The "CD8 positive T cells" refer to cells (CD8⁺CD4⁻) in which the surface antigen CD8 is positive among T cells. They are also called cytotoxic T cells. Since T cells can be recognized by whose surface antigens CD3 and CD45 being positive, CD8 positive T cells can be identified as the cells whose CD8, CD3 and CD45 are positive and whose CD4 is negative.

In an embodiment of the present invention, CD8 positive T cells can be produced by a method comprising a step in which CD4CD8 double positive T cells are cultured in a culture medium added with a corticosteroid.

In an embodiment of the present invention, the corticosteroid is preferably a glucocorticoid or a derivative thereof. Their examples include cortisone acetate, hydrocortisone, fludrocortisone acetate, prednisolone, triamcinolone, methylprednisolone, dexamethasone, betamethasone and beclomethasone dipropionate. Preferable corticosteroid is dexamethasone. Its concentration in a culture medium is, for example, 1 nM ∼ 100 nM.

In an embodiment of the present invention, the culture medium for producing CD8 positive T cells is not particularly limited. It can be prepared by using a medium useful for culturing animal cells as a minimal essential medium and adding a corticosteroid thereto. Examples of minimal essential media include Iscove's Modified Dulbecco's Medium (IMDM), Medium 199, Eagle's minimum essential medium (EMEM), αMEM medium, Dulbecco's modified Eagle's Medium (DMEM), Ham's F12 medium, RPMI 1640 medium, Fischer's medium, Neurobasal Medium (Life Technologies), and a mixed medium thereof. A medium may contain serum or may be serum-free. If necessary, a minimal essential medium may contain one or more substances, for example, albumin, insulin, transferrin, selenium, fatty acids, trace elements, 2-mercaptoethanol, thiolglycerol, lipids, amino acids, L-glutamine, non-essential amino acids, vitamins, growth factors, small molecule compounds, antibiotics, antioxidants, pyruvic acid, buffers, inorganic salts, cytokines, and the like.

In an embodiment of the present invention, preferably, the culture medium for producing CD8 positive T cells further contains an anti-CD3 antibody, a vitamin C, or a cytokine. Examples of the cytokines include IL-2 and IL-7.

In an embodiment of the present invention, the anti-CD3 antibody is not particularly limited as long as it specifically recognizes CD3, for example, an antibody produced from an OKT3 clone. The concentration of the anti-CD3 antibody in a culture medium is, for example, 10 ng/ml ∼ 1000 ng/ml.

In an embodiment of the present invention, vitamin Cs for producing CD8 positive T cells are, for example, those described above, and can be used under the same conditions as described above.

In an embodiment of the present invention, the concentration of a cytokine for producing CD8 positive T cells in a culture medium is, for example, 10 U/ml ∼ 1000 U/ml for IL-2 and 1 ng/ml ∼ 100 ng/ml for IL-7.

In an embodiment of the present invention, the temperature for culturing CD4CD8 double positive T cells for producing CD8 positive T cells is not particularly limited. However, for example, a temperature in the ranges of about 37°C ∼ about 42°C and about 37°C ∼ about 39°C is preferable. In addition, those skilled in the art can appropriately determine the culture period while monitoring the number of CD8 positive T cells, and the like. As long as CD8 positive T cells can be obtained, the number of days is not particularly limited, for example, at least 1 day or more, 2 days or more, 3 days or more, 4 days or more, 5 days or more, preferably 3 days.

The population of regenerated T cells obtained by the production method of the present invention is not particularly limited. Their examples include a population of αβ T cells, a population of γδ T cells, a population of helper T cells, a population of regulatory T cells, a population of cytotoxic T cells, a population of NK T cells, a population of tumor-infiltrating T cells, and the like.

In an embodiment of the present invention, the degree of genetic diversity maintained is preferably 30% or more, 40% or more and 50% or more of the population of genetically diverse T cells of raw materials.

In an embodiment of the present invention, the population of regenerated T cells produced is preferably used for T cell replacement therapy. In the present invention, the T cell replacement therapy refers to a therapy for replenishing T cells into a living body. In an embodiment of the present invention, preferable examples of the T cell replacement therapy include therapeutic methods by cell transplantation, such as infusion, intratumoral injection, arterial injection and portal vein injection of regenerated T cells, etc.

An embodiment of the present invention includes a population of regenerated T cells obtained via iPS cells, while maintaining the genetic diversity of a population of *in vivo* T cells, for example, a population of regenerated T cells obtained by the above method. The pharmaceutical composition containing the population of regenerated T cells of the present invention can be used for treating cancer subjects. An embodiment of the present invention includes a method for treating cancer using the pharmaceutical composition of the present invention. The pharmaceutical composition of the present invention may contain pharmaceutically acceptable additives. Examples of the additives include a cell culture medium, phosphate buffered saline, and the like.

An embodiment of the present invention includes a therapy which applies the population of regenerated T cells obtained by the production method of the present invention to T cell replacement therapy. In addition, an embodiment of the present invention includes a population of regenerated T cells obtained by the production method of the present invention for use in treatment with T cell replacement therapy.

An embodiment of the present invention includes all general embodiments and/or all combinations of specific embodiments listed in the above description.

The present invention will be described more specifically with reference to following examples. However, the scope of the present invention is not limited to these examples.

### EXAMPLES

### Detailed procedure of step (1)

Sampled population of T cells was activated by stimulation using magnetic beads labeled with CD3 and CD28 antibodies, and cultured for 2 days in RPMI-1640 medium (10% fetal bovine serum, containing 1% Penicillin-Streptomycin-Glutamine) added with 200 U/ml IL-2, 10 ng/ml IL-7 and 10 ng/ml IL-15.

### Detailed procedure of step (2) (reprogramming to iPS cells)

Subsequently, the T cells were collected in a 15 ml tube, suspended in about 250 µl of RPMI-1640 medium. Sendai virus, which contains four factors (Oct3/4, Sox2, Klf4, and c-Myc) required for reprogramming at different titers of MOI = 5 was then added to the medium using CytoTune-iPS 2.0 kit (ID Pharma, # DV-0304) and incubated at 37°C for 2 hours. After the incubation, the T cells were washed with RPMI-1640 medium to remove the virus from the medium.

Subsequently, in order to remove the magnetic beads, the beads and the cells were separated by pipetting. Then only the T cells suspended in the medium were collected by allowing the tube to stand still on a stand with a magnet.

The obtained T cells were seeded on a 6-cm dish coated with 0.5 µg/cm² of iMatrix-511 (Nippi, # 892011) and started culturing in RPMI-1640 medium using an incubator set at 37°C, 5% CO₂ and 5% O₂.

From the day after the start of culturing on the 6-cm dish, half of the cell-free culture supernatant was removed, and the same volume of medium for establishment, which is StemFit AK03N (Ajinomoto) containing 1 mM valproic acid, was added.

Thereafter, the culture was continued for 20 ∼ 40 days while exchanging half-volume medium every day in the same way until iPS cell colony in the 6-cm dish was obtained in a large number. When a certain number of colonies was obtained, the medium for establishment was switched to the medium for iPS cells (StemFit AK03N only) and the culture was continued.

### Detailed procedure of step (2) (culturing while maintaining genetic diversity of the established iPS cells)

When the iPS cell colony was large enough to be seen with naked eyes, the iPS cells were subcultured into a new 6-cm dish coated with iMatrix-511.

In the subculture, the iPS cells adhered to the dish were washed with PBS (-), added with TrypLeSelect (Life Technologies, A12859-01) and incubated for about 7 minutes. Accordingly, iPS cells were released from adhesion and dispersed into single cells. The cells were washed with the medium for iPS cells, and the number of cells was counted. Then, the dispersed cells were seeded on a 6-cm dish newly coated with iMatrix-511 at a density of 1,500 cells/cm². At this time, all the iPS cells collected were seeded on the new dish without discarding.

By inheriting all the cells collected, the scale of the culture gradually increases. Therefore, the cells were subcultured 3 to 5 times after the establishment. When the iPS cells were obtained in an enough amount required for T cell replacement therapy using iPS cells, the cells were cryopreserved with following procedure.

The iPS cells were collected in the same procedure as in the subculture, and then suspend in a cell freezing liquid such as a TC Protector (DS Pharma, # KBTCP001). The iPS cells were frozen by reducing the temperature to -80°C at a rate of -1°C/min. For stable and long-term storage, it is desirable to store iPS cells furthermore in liquid nitrogen (at -196°C).

### Detailed procedure of step (3)

The iPS cells obtained in step (2) were seeded on a 6-well plate (CORNING, # 3471) treated to ultra-low adhesion at 3×10⁵ ∼ 6×10⁵ cells/well (Day 0). 10 ng/ml BMP 4, 5 ng/ml bFGF, 15 ng/ml VEGF and 2 µM SB431542 were added to EB medium (StemPro34 added with 10 µg/ml human insulin, 5.5 µg/ml human transferrin, 5 ng/ml sodium selenite, 2 mM L-glutamine, 45 mM α-monothioglycerol and 50 µg/ml ascorbic acid). The culture was conducted for 5 days under hypoxic conditions (5% O₂) (Day 5).

Subsequently, 50 ng/ml SCF, 30 ng/ml TPO and 10 ng/ml F1t3L were added. The culture was further conducted for 5 ∼ 9 days (∼Day 14).

The hematopoietic progenitor cells obtained were cultured for 21 days (Day 35) in OP9 medium (added with 15% FBS, 2 mM L-glutamine, 100 U/ml penicillin, 100 ng/ml streptomycin, 55 µM 2-mercaptoethanol, 50 µg/ml ascorbic acid, 10 µg/ml human insulin, 5.5 µg/ml human transferrin and 5 ng/ml sodium selenite) added with 50 ng/ml SCF, 50 ng/ml IL-7, 50 ng/ml Flt3L, 100 ng/ml TPO, 15 µM SB203580 (Tocris Bioscience) and 30 ng/ml SDF-1α (PeproTech) on a 48-well plate coated with Fc-DLL4 (5 µg/ml, Sino Biological Inc.) and Retronectin (5 µg/ml, Takara Bio Inc.).

On Day 35, a fraction wherein CD45, CD3, CD4 and CD8 were all positive was separated using FACS, and CD4CD8 double positive cells (called DP cells) were obtained.

The CD4CD8 double positive cells obtained were cultured for 2 days (Day 37) on a 96-well plate in RPMI 1640 medium added with 15% fetal bovine serum, 500 ng/ml anti-CD3 antibody (eBioscience), 200 U/ml IL-2, 10 ng/ml IL-7 and 10 nM dexamethasone. The cells were washed with RPMI 1640 medium containing 15% fetal bovine serum to remove the antibody and cultured further for 5 days in RPMI 1640 medium added with 15% fetal bovine serum and 10 ng/ml IL-7. CD8 positive T cells were obtained (Day 42).

### Example 1

Using the above method, iPS cells were established from a population of genetically diverse T cells separated from a resected tumor of a lung cancer patient and used for induction of regeneration into genetically diverse T cells.

The genetic diversity of T cell receptors (TCRs) expressed by T cells is generally determined by DNA or RNA sequence analysis. It can also be detected easily by using antibody panels that specifically bind to each segment of Vβ chain in TCR.

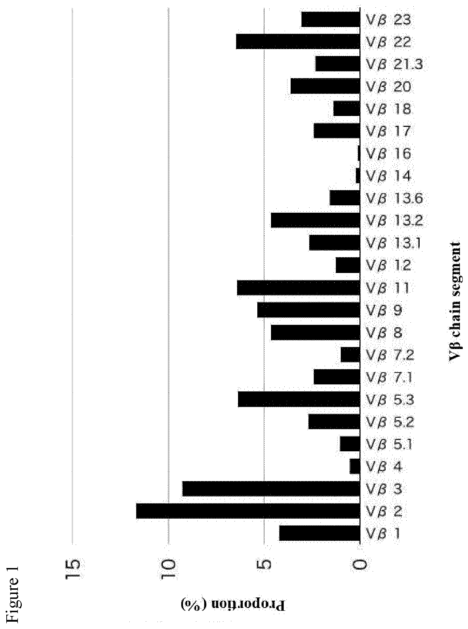

The TCR repertoire of a population of T cells separated from a resected tumor of a lung cancer patient was analyzed with a TCRVβ analysis kit (BECKMAN COULTER, # IM-3497) (Figure 1). The sample from the patient contained cells expressing each Vβ chain at a proportion of about 1 ∼ 13%, which were confirmed to be the population of genetically diverse T cells. These population of T cells were reprogrammed into iPS cells by the method described above.

iPS cells are pluripotent stem cells. They do not express T cell-related genes including TCR gene. That is, it is difficult to analyze the diversity of the TCR gene at undifferentiated iPS cell stage. The established iPS cells were subcultured so as not to lose genetic diversity and then induced to differentiate into T cells via hematopoietic progenitor cell stage.

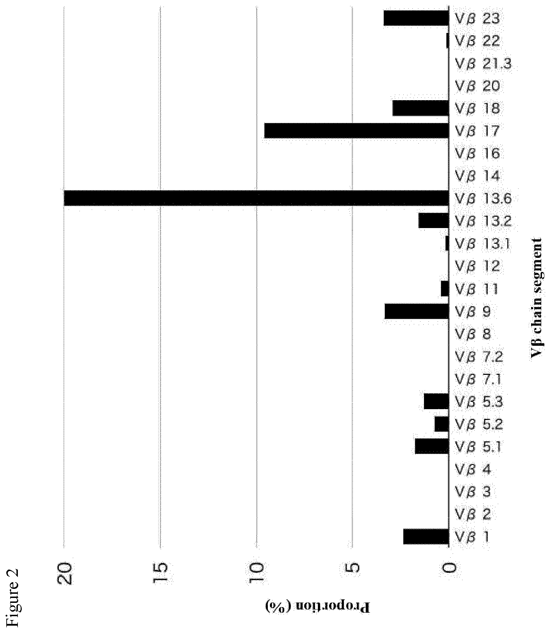

The repertoire of TCR expressed by the population of regenerated T cells was analyzed using the same kit described above (Figure 2). Due to the establishment of iPS cells and induction of T cell differentiation, the diversity of T cells expressing segments such as 2, 3, 4, 7.1, 7.2, 8, 12, 14, 16, 20, and 21.3 of Vβ was lost. Despite this, about half of the Vβ repertoire was conserved. The regenerated T cells were confirmed to be genetically diverse T cells.

## Claims

1. A method for producing a population of genetically diverse regenerated T cells via iPS cells, the method comprising
(1) obtaining a population of T cells that can recognize a target tissue or antigens from a sample of a population of genetically diverse T cells;
(2) reprogramming the obtained the population of T cells into iPS cells, culturing the iPS cells while maintaining genetic diversity; and
(3) producing a population of genetically diverse regenerated T cells from the cultured iPS cells.

2. The method according to claim 1, wherein the population of T cells in (1) is derived from a mammalian subject.

3. The method according to claim 1 or 2, wherein the population of T cells in (1) are tumor-infiltrating T cells.

4. The method according to claim 1 or 2, wherein the population of T cells in step (1) is derived from blood, lymph nodes or cavity fluid.

5. The method according to any one of claims 1-4, wherein (1) further comprising separating the proliferated T cells, which are activated by stimulation with an antigen protein or peptides.

6. The method according to any one of claims 1-5, comprising collecting iPS cells without cloning and subculturing the iPS cells in (2).

7. The method according to any one of claims 1-6, wherein the population of T cells obtained in step (3) is, a population of αβ T cells, a population of γδ T cells, a population of helper T cells, a population of regulatory T cells, a population of cytotoxic T cells, a population of NK T cells or a population of tumor-infiltrating T cells.

8. The method according to any one of claims 1-7, wherein the population of genetically diverse regenerated T cells obtained (3) is used for T cell replacement therapy.

9. A population of regenerated T cells obtained by the method according to any one of claims 1-8.

10. A population of regenerated T cells obtained via iPS cells that maintains the genetic diversity of the population of T cells present *in vivo.*

11. A pharmaceutical composition, comprising the population of regenerated T cells according to claim 9 or 10.

12. The pharmaceutical composition according to claim 11 for treating cancer subjects by autologous or allogeneic transplantation.

13. A method for treating cancers, which uses the pharmaceutical composition according to claim 11 or 12.
